(19) Europäisches Patentamt
European Patent Office
Office européen des brevets

(11) EP 1 683 513 B1

(12) # FASCICULE DE BREVET EUROPEEN

(45) Date de publication et mention de la délivrance du brevet:
**02.03.2011 Bulletin 2011/09**

(51) Int Cl.:
***A61K 8/39*** *(2006.01)* ***A61Q 1/06*** *(2006.01)*
***A61Q 19/00*** *(2006.01)* ***A61K 8/37*** *(2006.01)*

(21) Numéro de dépôt: **05292241.6**

(22) Date de dépôt: **24.10.2005**

(54) **Composition cosmétique contenant un ester d'alcool alcoxylé et une huile ester hydrocarbonée**

Kosmetische Zusammensetzung enthaltend einen Ester eines alkoxylierten Alkohols und einen Kohlenwasserstoff-Ester

Cosmetic composition comprising an ester of an alcoxylated alcohol and a hydrocarbon ester oil

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **30.12.2004 FR 0453265**

(43) Date de publication de la demande:
**26.07.2006 Bulletin 2006/30**

(73) Titulaire: **L'Oréal**
**75008 Paris (FR)**

(72) Inventeurs:
- **Ricard, Audrey**
  **75012 Paris (FR)**
- **Lebre, Caroline**
  **94320 Thiais (FR)**
- **Filippi, Vanina**
  **75013 Paris (FR)**

(74) Mandataire: **Le Coupanec, Pascale A.M.P. et al**
**Nony & Associés**
**3, rue de Penthièvre**
**75008 Paris (FR)**

(56) Documents cités:

| | |
|---|---|
| **EP-A- 1 454 622** | **WO-A-2004/052076** |
| **FR-A- 2 843 541** | **US-A- 5 814 313** |
| **US-A- 6 039 960** | **US-A1- 2002 192 249** |
| **US-A1- 2003 069 388** | **US-A1- 2004 180 032** |
| **US-A1- 2004 247 543** | **US-B1- 6 423 324** |

- **WANG; LEE: "The effect of formulation on the hardness and crystallization of emulsion lipsticks" JOURNAL OF THE SOCIETY OFCOSMETIC CHEMISTS, vol. 48, 1997, pages 41-50, XP009054033**
- **PUNTO, LOUIS L. ET AL: "Multifunctionality from a new polymeric ester" COSMETICS & TOILETRIES , 119(11), 53-56, 58 CODEN: CTOIDG; ISSN: 0361-4387, novembre 2004 (2004-11), XP009066085**

Remarques:
Le dossier contient des informations techniques présentées postérieurement au dépôt de la demande et ne figurant pas dans le présent fascicule.

EP 1 683 513 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention se rapporte à une composition cosmétique de maquillage et/ou de soin de la peau et/ou des lèvres contenant un ester d'alcool alcoxylé et d'acide carboxylique particulier et un ester hydrocarboné particulier,

La composition de l'invention peut en particulier constituer un produit de maquillage des lèvres, ou du corps pouvant être doté en outre de propriétés de soin. La composition de l'invention peut constituer notamment un rouge à lèvres ou un brillant à lèvres, un fard à joues ou à paupières, un produit pour tatouage, un eye-liner, un produit de bronzage artificiel de la peau,

La composition cosmétique selon l'invention présente avantageusement une tenue de la couleur très satisfaisante tout en étant brillante et confortable.

La tenue est particulièrement recherchée par les utilisateurs de produits cosmétiques. Des rouges à lèvres de tenue satisfaisante sont généralement des sticks contenant des huiles esters de faible poids moléculaire ou des huiles silicones. Afin d'augmenter la tenue de ces produits, les derniers concepts de formule s'appuient sur l'association d'une phase « brillante» et d'une phase « couleur et confort » rendues compatibles par un solvant volatil. Lors de l'application du produit sur les lèvres, le volatile s'évapore puis un phénomène de ségrégation se produit. Ce concept reste toutefois limité pour améliorer la tenue et le confort. En effet, le domaine de compatibilité des phases reste restreint et le taux d'isododécane ne peut être augmenter au détriment de la duretés des sticks et surtout du confort.

On a proposé des formules contenant des copolymères acrylates/acrytiques dispersés dans un solvant volatile tel que l'isododécane. Ces polymères forment un film sur les lèvres après évaporation du solvant. Il faut néanmoins de très fort taux de volatiles dans ces formules pour assurer la tenue de la composition cosmétique sur les matières kératiniques, au détriment du confort du dépôt sur les matières kératiniques.

Il a été trouvé, de façon surprenante, qu'un ester alcoxylé particulier permet d'obtenir des formules dont la brillance est élevée et dont le confort est équivalent à une formule classique tout en améliorant de façon importante la tenue de la couleur par rapport aux formules connues.

Certains esters alcoxylés ont déjà été utilisés dans des compositions cosmétiques.

Ainsi, la demande US 2002/0192249 décrit des compositions cosmétiques comprenant un ester d'acide monocarboxylique comprenant de 4 à 24 atomes de carbone, et d'alcool comprenant un groupe polypropoxyle et une chaîne alkyle comportant de 2 à 24 atomes de carbone.

Les préparations peuvent comporter, outre l'ester alcoxylé, de l'huile minérale ou de l'huile de paraffine. Ce document décrit également une composition anhydre contenant cet ester et un agent filmogène, et une composition contenant du PPG-3 Myristyl Ether Néoheptanoate. Plus précisément, le document donne des compositions de rouges à lèvres contenant cet ester polypropoxylé en association avec du polyisobutène hydrogéné ; des compositions de fond de teint en crème en émulsion contenant cet ester; des compositions de filtres solaires en émulsion contenant du laurate d'hexyle, du palmitate d'octyle, et du palmitate de cétyle en association avec cet ester.

Le document US 5.693.316 divulgue des compositions cosmétiques contenant un ester gras alcoxylé obtenu à partir d'un acide dicarboxylique ayant de 2 à 22 atomes de carbone, en particulier l'acide maléique, et d'un excès stoechiométrique de un ou plusieurs alcools gras polyalcoxylés comportant une chaîne alkyle comprenant de 14 à 22 atomes de carbone et un groupe polyalcoxyle. Les préparations peuvent comporter de l'huile minérale ou de l'huile de paraffine comme deuxième émollient. Ce document décrit également une composition anhydre contenant cet ester et un agent filmogène. L'ester alcoxylé est en particulier le Di-PPG-3 Myristyl maléate.

Le document US 6.476.254 divulgue des compositions cosmétiques contenant un ester d'acide dicarboxylique comprenant de 4 à 12 atomes de carbone et d'alcool gras polyalcoxylé dont la partie non alcoxylée comprend de 8 à 36 atomes de carbones. L'ester peut être le Di-PPG-3 Myristyl adipate. La composition peut être anhydre. Elle peut contenir de l'huile minérale ou de l'huile de paraffine.

La demande WO 2003/013439 a pour objet un ester d'acide dicarboxylique en C3-C21 ou tricarboxylique aliphatique en C4-C22, notamment de C3 à C9, et d'alcool gras polyalcoxylé comprenant un radical alkyle en C6-C30, notamment en C18-C22. Ce document décrit une composition cosmétique qui peut contenir de la vaseline, de l'huile minérale, des esters d'acides carboxylique aliphatiques et d'alcools aliphatiques comprenant de 18 à 40 atomes de carbone, un agent filmogène, ou un alcool gras tel que l'alcool cétylique.

Les documents US 5.302.377, US 5.455.025 et US 5.597.555 divulguent des compositions cosmétiques contenant un ester gras alcoxylé d'acide tricarboxylique, en particulier l'acide citrique, avec un excès stoechiométrique de un ou plusieurs alcools gras polyalcoxylés ayant des propriétés émollientes pour des préparations topiques. Les préparations contiennent une huile minérale comme deuxième émollient. Ce document décrit également l'association de cet ester avec un agent filmogène. L'ester est en particulier le Tri-PPG-3 Myristyl citrate.

Le document WO 052076 décrit des compositions cosmétiques contenant des esters mixtes d'alcools polyalcoxylés et d'alcools monohydriques avec des acides polycarboxyliques, notamment des acides dicarboxyliques. Ces compositions peuvent contenir un deuxième agent émollient tel que l'huile minérale ou la vaseline. Les esters mixtes décrits peuvent être formulés en association avec un composé filmogène.

La présente invention a pour objet une composition cosmétique telle que définie en revendication 1

L'invention a encore pour objet l'utilisation cosmétique d'une telle composition

L'invention a encore pour objet un procédé de soin et/ou de maquillage de la peau et/ou des lèvres comprenant l'application sur la peau et/ou les lèvres d'une telle composition

**ESTER ALCOXYLE**

La composition selon l'invention contient au moins un ester d'alcool alcoxylé et d'acide carboxylique, appelé par la suite ester alcoxylé, choisi parmi

- l'Octyldodécyl PPG-3 Myristyl Ether Dilinoléate commercialisé sous la référence LIQUIWAX polyEFA par la société ARCH CHEMICAL de formule

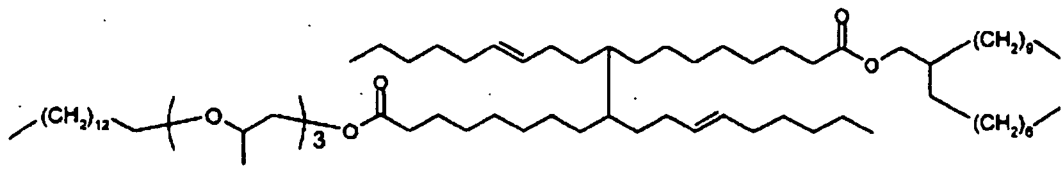

- le Stéaryl PPG-3 Myristyl Ether Dilinoléate commercialisé sous la référence liquiwax polyIPL par la société ARCH CHEMICAL, et
- l'isostéaryl PPG-4 Butyloctyl Ether Dilinoléate.

Selon un mode de réalisation, l'ester alcoxylé est l'Octyldodécyl PPG-3 Myristyl Ether Dilinoléate.

Selon un autre mode de réalisation, l'ester alcoxylé est l'isostéaryl PPG-4 Butyloctyl Ether Dilinoléate.

L'ester alcoxylé est avantageusement présent dans la composition à raison de 1 à 99 % en particulier, notamment de 2 à 60 % en poids, en particulier de 5 à 40 % et plus particulièrement de 10 à 35 % en poids par rapport au poids total de la composition.

**ESTER HYDROCARBONE**

Par « ester hydrocarboné», on entend un composé différent de l'ester alcoxylé décrit précédemment et comprenant au moins une fonction ester COO. s'agit d'un monoester choisi parmi :

les esters de l'acide néopentanoïque comme le néopentanoate d'isodécyle, le néopentanoate d'isotridécyle, le néopentanoate d'isostéaryle, le néopentanoate d'octyldodécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, l'érucate d'oléyle et leurs mélanges.

L'isononanoate d'isononyle, l'érucate d'oléyle, le néopentanoate d'octyldodécyle et leurs mélanges conviennent tout particulièrement à la réalisation de l'invention.

Cet ou ces ester(s) hydrocarboné(s) peut(peuvent) être utilisé(s) dans la composition à raison de 5 à 90 %, notamment de 10 à 60 % et en particulier de 20 à 50 % en poids par rapport au poids total de la composition.

**TENUE**

La composition est apte à former un dépôt ayant un indice de tenue supérieur ou égal à 30 %, de préférence supérieur ou égal à 40 %, de préférence supérieur ou égal à 45 %, de préférence supérieur ou égal à 50 %, voire supérieur ou égal à 60 %, voire même supérieur ou égal à 65 %.

De façon avantageuse, l'ester hydrocarboné est tel, que lorsqu'il est en quantité suffisante dans la composition, celle-ci est apte à former un dépôt dont la tenue est supérieure ou égale à 30%.

L'indice de tenue du dépôt obtenu avec la composition selon l'invention peut être déterminé selon le protocole de mesure décrit ci-après.

On prépare un support (rectangle de 40 mm X 70 mm) constitué d'un revêtement acrylique (adhésif acrylique hypoallergénique sur film polyéthylène vendu sous la dénomination BLENDERME ref FH5000-55113 par la société 3M Santé) collé sur une couche de mousse de polyéthylène adhésif sur la face opposée à celle sur laquelle est fixé le

sparadrap (couche de mousse vendue sous la dénomination RE40X70EP3 de la société JOINT TECHNIQUE LYONNAIS IND).

On mesure à l'aide d'un colorimètre MINOLTA CR 300 la couleur $L^*_0a^*_0b^*_0$ du support , côté face revêtement acrylique.

On préchauffe le support ainsi préparé sur une plaque chauffante maintenue à la température de 40 °C pour que la surface du support soit maintenue à une température de 33°C ± 1 °C.

Tout en laissant le support sur la plaque chauffante, on applique la composition sur toute la surface non adhésive du support (c'est-à-dire sur la surface du revêtement acrylique) en l'étalant à l'aide d'un pinceau pour obtenir un dépôt de la composition d'environ 15 µm puis on laisse sécher pendant 10 minutes.

Après séchage, on mesure la couleur L*a*b* du film ainsi obtenu.

On détermine alors la différence de couleur ΔE1 entre la couleur du film par rapport à la couleur du support nu par la relation suivante.

$$\Delta E1 = \sqrt{(L^*-L_o^*)^2 + (a^* - a_o^*)^2 + (b^* - b_o^*)^2}$$

Le support est ensuite collé par sa face adhésive (face adhésive de la couche de mousse) sur une enclume d'un diamètre de 20 mm et munie d'un pas de vis. Une éprouvette de l'ensemble support/dépôt est ensuite découpée à l'aide d'un emporte- pièce d'un diamètre de 18 mm. L'enclume est ensuite vissée sur une presse (STATIF MANUEL IMADA SV-2 de la société SOMECO) équipée d'un dynanomètre (IMADA DPS-20 de la société SOMECO).

Sur un papier blanc pour photocopieuse de grammage 80g/m2, on dessine une bande de 33 mm de largeur et 29,7 cm de longueur, on trace un premier trait à 2 cm du bord de la feuille, puis un deuxième trait à 5 cm du bord de la feuille, les premier et deuxième traits délimitant ainsi une case sur la bande ; puis on dispose une première marque et une deuxième marque situées dans la bande respectivement aux repères 8 cm et 16 cm du deuxième trait . On place sur la première marque 20 µl d'eau et sur la deuxième marque 10 µl d'huile de tournesol raffinée (vendue par la société LESIEUR).

Le papier blanc est placé sur le socle de la presse puis on presse l'éprouvette placée sur la case de la bande de papier à une pression d'environ 300 g/cm$^2$ exercée pendant 30 secondes. Puis on relève la presse et on place à nouveau l'éprouvette juste après le deuxième trait (donc à côté de la case), on effectue à nouveau une pression d'environ 300 g/cm$^2$ et on déplace, de manière rectiligne dès le contact effectué, le papier avec une vitesse de 1 cm/s, sur toute la longueur de la bande de telle sorte que l'éprouvette traverse les dépôts d'eau et d'huile.

Après retrait de l'éprouvette, une partie du dépôt a transféré sur le papier. On mesure alors la couleur L*', a*', b*' du dépôt resté sur l'éprouvette.

On détermine alors la différence de couleur ΔE2 entre la couleur du dépôt resté sur l'éprouvette par rapport à la-couleur du support nu par la relation suivante.

$$\Delta E2 = \sqrt{(L^{*\prime}-L_o^*)^2 + (a^{*\prime} - a_o^*)^2 + (b^{*\prime} - b_o^*)^2}$$

L'indice de tenue de la composition, exprimée en pourcentage, est égal au rapport :

$$100 \times \Delta E2 / \Delta E1$$

La mesure est effectuée sur 6 supports à la suite et l'indice de tenue correspond à la moyenne des 6 mesures obtenues avec les 6 supports.

<u>PATEUX</u>

La composition peut également contenir en outre au moins un composé pâteux.

La composition est avantageusement exempte de lanoline ou de dérivés de lanoline.

A titre illustratif des dérivés de lanoline utilisés conventionnellement, on peut notamment citer la lanoline liquide,

la lanoline réduite, la lanoline purifiée par adsorption, la lanoline acétylée, la cire de lanoline oxypropylénée (5 OP), l'acétate de lanoline liquide, l'hydroxylanoline, la polyoxyéthylène-lanoline, l'acide gras de lanoline, l'acide gras de lanoline dure, les esters de cholestéryle d'acide gras de lanoline, l'alcool de lanoline, l'acétate de l'alcool de lanoline, le lanolate d'isopropyle et autres.

Les lanolines présentent l'inconvénient d'être sensibles à la chaleur et aux ultraviolets. Elles ont tendance à s'oxyder avec un dégagement d'odeur désagréable, et leur couleur très jaune empêche de les utiliser dans des bases de soin non pigmentées et les bases incolores, ce qui limite leur utilisation dans les compositions cosmétiques.

Le demandeur a découvert les esters alcoxylés décrits précédemment sont de bons substituts de la lanoline ou de ses dérivés.

Par "pâteux" au sens de la présente invention, on entend désigner un composé gras lipophile, à changement d'état solide/liquide réversible, et comportant à la température de 23°C une fraction liquide et une fraction solide. On entend également par « pâteux », le polylaurate de vinyle.

Le composé pâteux est avantageusement choisi parmi :

- la lanoline et ses dérivés,
- les composés fluorés polymères ou non,
- les composés siliconés polymères ou non,
- les polymères vinyliques, notamment:
- les homopolymères d'oléfines
- les copolymères d'oléfines
- les homopolymères et copolymères de diènes hydrogénés
- les oligomères linéaires ou ramifiés, homo ou copolymères de (méth)acrylates d'alkyles ayant de préférence un groupement alkyle en $C_8$-$C_{30}$
- les oligomères homo et copolymères d'esters vinyliques ayant des groupements alkyles en $C_8$-$C_{30}$
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100,}$ de préférence en $C_2$-$C_{50}$,
- les esters,

et leurs mélanges.

Parmi les polyéthers liposolubles, on préfère en particulier les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les pâteux esters, on préfère notamment :

- les esters d'un glycérol oligomère, notamment les esters de diglycérol, en particulier les condensats d'acide adipique et de glycérol, pour lesquels une partie des groupes hydroxyle des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en $C_4$-$C_{50}$ et un diol ou un polyol en $C_2$-$C_{50}$, différent du polyester décrit précédemment,
- les esters aliphatiques d'ester résultant de l'estérification d'un ester d'acide hydroxycarboxylique aliphatique par un acide monocarboxylique aliphatique; et leurs mélanges, comme
    - l'ester résultant de la réaction d'estérification de l'huile de ricin hydrogénée avec l'acide isostéarique dans les proportions 1 pour 1 (1/1) ou monoisostéarate d'huile de ricin hydrogénée,
    - l'ester résultant de la réaction d'estérification de l'huile de ricin hydrogénée avec l'acide isostéarique dans les proportions 1 pour 2 (1/2) ou le diisostéarate d'huile de ricin hydrogénée,
    - l'ester résultant de la réaction d'estérification de l'huile de ricin hydrogénée avec l'acide isostéarique dans les proportions 1 pour 3 (1/3) ou triisostéarate d'huile de ricin hydrogénée,
    - et leurs mélanges.

Parmi les composés pâteux d'origine végétale, on choisira de préférence un mélange de stérols de soja et de pentaérythritol oxyéthyléné (50E) oxypropyléné (5 OP), commercialisé sous la référence Lanolide par la société VEVY.
Le composé pâteux représente de préférence 1 à 99%, mieux 1 à 60%, mieux 2 à 30% et mieux encore 5 à 15% en poids de la composition.

## COLORANT

Avantageusement, la composition de l'invention peut comprendre, en outre, au moins une matière colorante qui peut être choisie parmi les colorants hydrosolubles ou liposolubles et les matières pulvérulentes, tels que, les pigments, les nacres et les paillettes. Cette matière colorante peut représenter de 0,001 à 98 %, de préférence de 0,5 à 85% et mieux de 1 à 60 % du poids total de la composition.
Les colorants sont de préférence des colorants liposolubles, bien que les colorants hydrosolubles puissent être utilisés. Les colorants liposolubles son par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).
Pour une composition sous forme de pâte ou coulée telle que les rouges à lèvres ou les produits de maquillage du corps, on utilise en général de 0,5 à 50% de matière colorante, de préférence de 2 à 40 % et mieux de 5 à 30%, par rapport au poids total de la composition.
Par pigments, il faut comprendre des particules blanches ou colorées, minérales ou organiques, insolubles dans la ou les huiles de la composition, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.
Les pigments peuvent être présents dans la composition à raison de 0,05 à 30 % du poids de la composition finale, et de préférence à raison de 2 à 20 %. Comme pigments minéraux utilisables dans l'invention, on peut citer les oxydes de titane, de zirconium ou de cérium-ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.
Les nacres peuvent être présentes dans la composition à raison de 0,001 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré. La composition contient avantageusement des pigments goniochromatiques, par exemple des pigments multicouches interférentiels, et/ou des pigments réfléchissants. Ces deux types de pigments sont décrits dans la demande FR0209246.

## CHARGES

La composition peut contenir au moins une charge qui peut être présente à raison de 0,001 à 35 % du poids total de la composition, de préférence 0,5 à 15 %.
On peut notamment citer

- le talc, le mica, le kaolin, l'amidon
- les poudres de Nylon® (Orgasol notamment)
- les poudres de polyéthylène,
- les poudres de polytétrafluoroéthylène (Téflon®),
- le nitrure de bore,
- des microsphères de copolymères telles que l'Expancel® (Nobel Industrie),
- le Polytrap® 603 (Dow Corning),
- le Polypore® L 200 (Chemdal Corporation)
- les microbilles de résine de silicone (Tospearl ® de Toshiba, par exemple),
- les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass
- les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI.

La charge peut être par exemple une charge dont la granulométrie moyenne est inférieure à 100 μm, notamment comprise entre 1 et 50 μm, par exemple entre 4 et 20 μm.
La charge peut être de toute forme, essentiellement sphérique ou sous la forme de plaquettes.

CIRE

La composition peut contenir, en outre, au moins un corps gras choisi parmi les cires, les corps gras pâteux et leurs mélanges. Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.
Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.
Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba : les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.
La composition selon l'invention contient avantageusement de la cire de polyéthylène de masse moléculaire en poids comprise entre 300 et 700, notamment égale à 500 g/mol.
A titre indicatif, la cire peut représenter de 0,01 à 50 %, de préférence de 2 à 40 %, et mieux de 5 à 30 % du poids total de la composition.

HUILE NON VOLATILE

La composition peut également comprendre en outre au moins une huile non volatile, autre que l'ester d'alcool alcoxylé, et autre que l'ester hydrocarboné décrits précédemment. L'huile non volatile peut être choisie parmi:

- les huiles hydrocarbonées d'origine animale telles que le perhydrosqualène ;
- les huiles hydrocarbonées végétales telles que les triglycérides liquides d'acides gras ayant de 4 à 10 atomes de carbone comme les triglycérides des acides heptanoïque ou octanoïque ou l'huile de jojoba ;
- les hydrocarbures linéaires ou ramifiés, d'origine minérale ou synthétique tels que les huiles de paraffine et leurs dérivés, la vaseline ;
- les alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones (telles que la phényl triméthicone vendue sous le nom commercial DC556 par Dow Coming), les phényl diméthicones, les phényl triméthylsiloxy diphényl siloxanes, les diphényl diméthicones, les diphényl méthyldiphényl trisiloxanes, les 2-phényl éthyl triméthyl-siloxysilicates ;
- les acides gras ayant de 12 à 26 atomes de carbone comme l'acide oléïque
- et leurs mélanges.

Huile non volatile de masse moléculaire élevée

Selon un mode de réalisation, la composition contient une huile non volatile de masse moléculaire élevée, par

exemple comprise entre 650 à 10000 g/mol.

La composition selon l'invention contient avantageusement de 2 à 30 %, de préférence de 5 à 25%, de 5 à 15% d'au moins une huile de masse molaire allant de 650 à 10000 g/mol, et de préférence allant de 900 et 7500 g/mol.

Ainsi, l'huile de masse moléculaire allant de 650 à 10000 g/mol peut être choisie parmi

- les polybutylènes tels que L'INDOPOL H-100 (de masse molaire ou MM=965 g/mol), L'INDOPOL H-300 (MM=1340 g/mol), L'INDOPOL H-1500 (MM=2160g/mol) commercialisés ou fabriqués par la société AMOCO,
- les polyisobutylènes hydrogénés tels que le PANALANE H-300 E commercialisés ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol).
- les polydécènes et les polydécènes hydrogénés tels que le PURESYN 150 (MM=9200 g/mol) commercialisé par la société MOBIL CHEMICALS,
- les copolymères de la vinylpyrrolidone tels que le copolymère vinylpyrrolidone/1-héxadécène, ANTARON V-216 commercialisé ou fabriqué par la société ISP (MM=7300 g/mol),
- les esters tels que :

  a) les esters d'acides gras linéaires ayant un nombre total de carbone allant de 35 à 70 comme le tétrapélargonate de pentaérythrityle (MM=697,05 g/mol),
  b) les esters hydroxylés tels que le triisostéarate de polyglycérol-2 (MM=965,58 g/mol),
  c) les esters aromatiques tels que le tridécyl trimellitate (MM=757,19 g/mol),
  d) les esters d'alcool gras ou d'acides gras ramifiés en $C_{24}$-$C_{28}$ tels que ceux décrits dans la demande EP-A-0 955 039, et notamment le citrate de triisoarachidyle (MM=1033,76 g/mol), le tétraisononanoate de pentaérythrityle (MM=697.05g/mol), le triisostéarate de glycéryle (MM=891.51 g/mol), le tri décyl-2tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538.66 g/mol),
  e) les esters et polyesters de dimère diol, tels que les esters de dimère diol et d'acide gras , et les esters de dimère diols et de diacide.
  Les esters de dimère diol et d'acide mono-carboxylique peuvent êtres obtenus à partir d'acide mono-carboxylique comprenant de 4 à 34 atomes de carbone notamment de 10 à 32 atomes de carbone, lesquels acides sont linéaires, ramifiés, saturés ou insaturés.
  A titre illustratif des exemples d'acide mono-carboxylique convenant à l'invention, on peut notamment citer les acides gras.
  Les esters de dimère diol et d'acide dicarboxylique peuvent être obtenus à partir d'un dimére diacide dérivé en particulier de la dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18}$.
  Selon une variante particulière, il s'agit plus particulièrement du dimère diacide dont dérive également le dimère diol à estérifier.
  Les esters de dimère diol peuvent être obtenus à partir d'un dimère diol produit par hydrogénation catalytique d'un dimère diacide tel que décrit précédemment, par exemple le diacide dilinoléique hydrogéné.
  A titre illustratif des esters de dimère diol, on peut notamment citer les esters de diacides dilinoléiques et de dimères diols dilinoléiques commercialisés par la société NIPPON FINE . CHEMICAL sous la dénomination commerciale LUSPLAN DD-DA5® et DD-DA7®.

- les huiles siliconées telles que les silicones phénylées comme la BELSIL PDM 1000 de la société WACKER (MM=9000 g/mol),
- les huiles d'origine végétale telles que l'huile de sésame (820,6 g/mol),
- et leurs mélanges.

Les huiles non volatiles peuvent représenter de 0,001 à 90 % du poids total de la composition, de préférence de 0,05 à 60 % et mieux de 1 à 35 %.

<u>HUILE VOLATILE</u>

On peut inclure une ou plusieurs huiles volatiles dans la composition.
Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression

atmosphérique, en particulier ayant une pression de vapeur allant de 0,13 Pa à 40 000 Pa ($10^{-3}$ à 300 mm de Hg), et de préférence allant de 1,3 Pa à 13 000 Pa (0,01 à 100 mm de Hg), et préférentiellement allant de 1,3 Pa à 1 300 Pa (0,1 à 10 mm de Hg).

En outre, l'huile volatile a généralement un point d'ébullition, mesuré à pression atmosphérique, allant de 150°C à 260 °C, et de préférence allant de 170 °C à 250 °C.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Par huile siliconée; on entend une huile contenant au moins un atome de silicium, et notamment contenant des groupes Si-O.

Par huile fluorée, on entend une huile contenant au moins un atome de fluor.

L'huile volatile peut être siliconée ou hydrocarbonée.

L'huile volatile siliconée utilisable dans l'invention peut être choisie parmi les huiles siliconées ayant un point éclair allant de 40°C à 102 °C, de préférence ayant un point éclair supérieur à 55 °C et inférieur ou égal à 95 °C, et préférentiellement allant de 65 °C à 95 °C.

Comme huiles siliconées volatiles utilisables dans l'invention, on peut citer les silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl-cyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huiles siliconées volatiles utilisables dans l'invention, on peut citer tes silicones décrites dans la demande FR0304259 non publiée.

L'huile volatile hydrocarbonée utilisable dans l'invention peut être choisie parmi les huiles hydrocarbonées ayant un point éclair allant de 40°C à 102 °C, de préférence allant de 40 °C à 55 °C, et préférentiellement allant de 40 °C à 50 °C.

Comme huile volatile hydrocarbonée, on peut citer les huiles volatiles hydrocarbonées ayant de 8 à 16 atomes de carbone et leurs mélanges, et notamment les alcanes ramifiés en $C_8C_{16}$ comme les iso-alcanes (appelées aussi isoparaffines) en $C_8$-$C_{18}$, l'isododécane, l'isodécane, l'isohexadécane et par exemple les huiles vendues sous les noms commerciaux d'Isopars ou de Permetyls, les esters ramifiés en $C_8$-$C_{18}$ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

L'huile volatile représente par exemple de 5 à 97,5 % du poids total de la composition, et mieux de 10 à 75 %, de préférence entre 20 et 50% du poids total de la composition.

L'huile volatile représente de préférence 20 à 50 % en poids de la composition, de préférence de 30 à 40%, de préférence 35% environ.

ADDITIFS

La composition de , l'invention peut comprendre, en outre, tout additif complémentaire usuellement utilisé dans le domaine cosmétique, tel que de l'eau, des antioxydants, des polymères filmogènes, des gommes de silicone, des conservateurs, des neutralisants, des plastifiants, des gélifiants lipophiles ou des composés non aqueux liquides, des gélifiants de phase aqueuse, des dispersants, des actifs cosmétiques. Ces additifs, à l'exception de l'eau qui peut représenter de 0 à 70 % et par exemple de 1 à 50 et mieux de 1 à 10 % du poids total de la composition, peuvent être présents dans la composition à raison de 0.0005 à 20% du poids total de la composition et mieux de 0,001 à 10%.

Comme actif cosmétique utilisable dans invention, on peut citer les vitamines A, E, C, $B_3$, F, les provitamines comme le D-panthénol, la glycérine, les actifs apaisants comme l'$\alpha$-bisabolol, l'aloe vera, l'allantoïne, les extraits de plantes ou les huiles essentielles, les agents protecteurs ou restructurants comme les céramides, les actifs "fraîcheur" comme le menthol et ses dérivés, les émollients (beurre de cacao, diméthicone), les hydratants (arginine PCA), les actifs antirides, les acides gras essentiels, les filtres solaires, et leurs mélanges.

Selon un mode de réalisation, la composition de l'invention comprend un ingrédient cosmétique choisi parmi les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les fibres, ou leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

GALENIQUES

Les applications des compositions selon l'invention sont multiples et concernent l'ensemble des produits cosmétiques colorés ou non et plus particulièrement les rouges à lèvres.

La composition de l'invention peut se présenter sous la forme de composition solide, compactée ou coulée notamment en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

Elle peut se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. De préférence, elle se présente sous forme de composition à phase continue huileuse et notamment anhydre ; dans ce cas, elle peut contenir une phase aqueuse à un taux inférieur à 5 %.

Selon un mode de réalisation, la composition de l'invention se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre, de stick ou de solide coulé.

Selon un mode de réalisation, elle se présente sous forme anhydre.

La composition selon l'invention peut se présenter sous la forme d'une composition, colorée ou non, de soin de la peau ou sous forme d'une composition de protection solaire. Si elle contient des actifs cosmétiques, elle peut alors être utilisée comme base de soin ou de traitement non thérapeutique pour la peau comme les mains ou le visage ou pour les lèvres (baumes à lèvres, protégeant les lèvres du froid et/ou du soleil et/ou du vent), produit de bronzage artificiel de la peau.

La composition de l'invention peut également se présenter sous la forme d'un produit de maquillage coloré de la peau, en particulier du visage comme un blush, un fard à joues ou à paupières, de maquillage du corps comme un produit de tatouage semi-permanent ou de maquillage des lèvres comme un rouge ou un brillant à lèvres, présentant éventuellement des propriétés de soin ou de traitement non thérapeutique,

De préférence, la composition selon l'invention se présente sous forme d'un rouge à lèvres ou d'un brillant à lèvres.

Bien entendu la composition de l'invention doit être physiologiquement acceptable (en particulier cosmétiquement acceptable), à savoir non toxique et susceptible d'être appliquée sur la peau, les phanères ou les lèvres d'êtres humains.

Par « cosmétiquement acceptable », on entend agréable de goût, de toucher, d'aspect et/ou d'odeur, applicable plusieurs jours pendant plusieurs mois.

La composition selon l'invention peut être fabriquée par les procédés connus, généralement utilisés dans le domaine cosmétique.

Les exemples qui suivent ont pour but d'illustrer de manière non limitative l'objet de la présente invention. Les quantités sont données en pourcentage massique.

**Exemple 1 : Stick de rouge à lèvres**

| **Nom chimique** | **Quantité en gramme** |
|---|---|
| Ethers de dodécanediol et de polyéthylène glycol | 10.3 |
| Octyldodecyl PPG-3 myristyl ether dimer dilinoleate (Liquiwax POLYEFA) | 22.15 |
| Néopentanoate d'isostéaryle | 30.3 |
| Stearoyl stéarate d'isocétyle | 20.6 |
| Polylaurate de vinyle | 16.5 |
| Ditertiobutyl 4-hydroxytoluène | 0.15 |
| Copolymère acétate de vinyle / stéarate d'allyle | 7.5 |
| Poly isobutène hydrogéné | 10 |
| Copolymère polybutène/polyisobutène | 10 |

(suite)

| Nom chimique | Quantité en gramme |
|---|---|
| Cire de polyéthylène | 3.4 |
| Cire microcristalline | 2.55 |
| Stéarate d'octacosanyle | 4.25 |
| Esters d'acides et alcools linéaires | 3.4 |
| Oxyde de titane rutile traité alumine/silice/tri-méthylolpropane | 0.27 |
| Laque d'aluminium de sel di-sodique de phloxine B sur elumine, benzoate d'aluminium | 0.66 |
| Sel de calcium du rouge lithol B | 0.16 |
| Oxydes de fer brun, jaune | 0.64 |
| Oxyde de fer noir | 0.66 |
| Microsphères de silice poreuse enrobées de polydiméthylsiloxane | 5 |
| parfum | 0.4 |

La brillance moyenne de ce rouge à lèvres T0h et T5h mesurée à 60°, selon la méthode décrite précédemment, sont égales à 79 sur 100.

## Revendications

1. Composition cosmétique de maquillage et/ou de soin de la peau et/ou des lèvres comprenant au moins un ester d'alcool alcoxylé et d'acide carboxylique et au moins un ester hydrocarboné différent de l'ester précédent, ladite composition étant apte à former un dépôt ayant un indice de tenue supérieur ou égal à 30 %, ledit ester d'alcool alcoxylé et d'acide carboxylique étant choisi parmi l'Octyldodécyl PPG-3 Myristyl Ether Dilinoléate, le Stéaryl PPG-3 Myristyl Ether Dilinoléate et l'Isostéaryl PPG-4 Butyloctyl Ether Dilinoléate, et ledit ester hydrocarboné étant un monoester choisi parmi les esters de l'acide néopentanoïque comme le néopentanoate d'isodécyle, le néopentanoate d'isotridécyle, le néopentanoate d'isostéaryle, et le néopentanoate d'octyldodécyle, les esters de l'acide isononanoïque comme l'isononanoate d'isononyle, l'isononanoate d'octyle, l'isononanoate d'isodécyle, l'isononanoate d'isotridécyle, et l'isononanoate d'isostéaryle, l'érucate d'oléyle et leurs mélanges.

2. Composition selon la revendication 1, **caractérisée en ce que** ledit monoester est choisi parmi le néopentanoate d'octyldodécyle, l'isononanoate d'isononyle, l'érucate d'oléyle et leurs mélanges.

3. Composition selon la revendication 1 ou 2, **caractérisée en ce que** l'ester alcoxylé est l'Octyldodécyl PPG-3 Myristyl Ether Dilinoléate.

4. Composition selon la revendication 1 ou 2, **caractérisée en ce que** l'ester alcoxylé est l'isostéaryl PPG-4 Butyloctyl Ether Dilinoléate.

5. Composition selon l'une des revendications précédentes, **caractérisée en ce que** l'ester hydrocarboné est tel, que lorsqu'il est en quantité suffisante dans la composition, ladite composition est apte à former un dépôt dont la tenue est supérieure ou égale à 30%.

6. Composition selon l'une quelconque des revendications précédentes, **caractérisée par le fait qu'**elle est apte à former un dépôt ayant un indice de tenue supérieur ou égal à 40 %.

7. Composition selon la revendication précédente, **caractérisée par le fait qu'**elle est apte à former un dépôt ayant un indice de tenue supérieur ou égal à 50 %, voire supérieure à 60%, voire même supérieure ou égale à 65%.

8. Composition selon l'une quelconque des revendications précédentes, **caractérisée en ce qu'**elle comprend, en outre, une ou des matières colorantes choisies parmi les colorants hydrosolubles ou liposolubles et les matières

colorantes pulvérulentes, tels que les pigments, les nacres et les paillettes.

**9.** Composition selon l'une quelconque des revendications précédentes, **caractérisée par le fait qu'**elle comprend en outre au moins un corps gras choisi parmi les cires, les corps gras pâteux et leurs mélanges.

**10.** Composition selon l'une quelconque des revendications précédentes, **caractérisée par le fait qu'**elle comprend un ingrédient cosmétique choisi parmi les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les fibres, ou leurs mélanges.

**11.** Composition cosmétique selon l'une quelconque des revendications précédentes, **caractérisée en ce qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre, de stick ou de solide coulé.

**12.** Composition cosmétique selon l'une quelconque des revendications 1 à 11, **caractérisée en ce qu'**elle se présente sous forme anhydre.

**13.** Composition selon l'une des revendications 1 à 11, **caractérisée en ce qu'**il s'agit d'un produit de maquillage des lèvres.

**14.** Utilisation cosmétique d'une composition selon l'une des revendications 1 à 12.

**15.** Procédé de maquillage et/ou de soin de la peau et/ou des lèvres comprenant l'application sur la peau, les lèvres d'au moins une composition selon l'une quelconque des revendications 1 à 12.

## Claims

**1.** Cosmetic composition for making up and/or caring for the skin and/or the lips, comprising at least one ester of an alkoxylated alcohol and of a carboxylic acid and at least one hydrocarbon-based ester different from the preceding ester, the said composition being capable of forming a deposit with a remanence index of greater than or equal to 30%, the said ester of an alkoxylated alcohol and of a carboxylic acid being chosen from octyldodecyl PPG-3 myristyl ether dilinoleate, stearyl PPG-3 myristyl ether dilinoleate and isostearyl PPG-4 butyloctyl ether dilinoleate, and the said hydrocarbon-based ester being a monoester chosen from neopentanoic acid esters such as isodecyl neopentanoate, isotridecyl neopentanoate, isostearyl neopentanoate and octyldodecyl neopentanoate, isononanoic acid esters such as isononyl isononanoate, octyl isononanoate, isodecyl isononanoate, isotridecyl isononanoate and isostearyl isononanoate, and oleyl erucate, and mixtures thereof.

**2.** Composition according to Claim 1, **characterized in that** the said monoester is chosen from octyldodecyl neopentanoate, isononyl isononanoate and oleyl erucate, and mixtures thereof.

**3.** Composition according to Claim 1 or 2, **characterized in that** the alkoxylated ester is octyldodecyl PPG-3 myristyl ether dilinoleate.

**4.** Composition according to Claim 1 or 2, **characterized in that** the alkoxylated ester is isostearyl PPG-4 butyloctyl ether dilinoleate.

**5.** Composition according to one of the preceding claims, **characterized in that** the hydrocarbon-based ester is such that when it is in sufficient amount in the composition, the said composition is capable of forming a deposit with a remanence of greater than or equal to 30%.

**6.** Composition according to any one of the preceding claims, **characterized in that** it is capable of forming a deposit with a remanence index of greater than or equal to 40%.

**7.** Composition according to the preceding claim, **characterized in that** it is capable of forming a deposit with a remanence index of greater than or equal to 50%, or even greater than 60%, or even greater than or equal to 65%.

8. Composition according to any one of the preceding claims, **characterized in that** it also comprises one or more dyestuffs chosen from water-soluble or liposoluble dyes and pulverulent dyestuffs, such as pigments, nacres and glitter flakes.

9. Composition according to any one of the preceding claims, **characterized in that** it also comprises at least one fatty substance chosen from waxes and pasty fatty substances, and mixtures thereof.

10. Composition according to any one of the preceding claims, **characterized in that** it comprises a cosmetic ingredient chosen from vitamins, thickeners, gelling agents, trace elements, softeners, sequestrants, fragrances, acidifying or basifying agents, preserving agents, sunscreens, surfactants, antioxidants and fibres, or mixtures thereof.

11. Cosmetic composition according to any one of the preceding claims, **characterized in that** it is in the form of a suspension, a dispersion, a solution, a gel, an emulsion, especially an oil-in-water (O/W) or water-in-oil (W/O) emulsion, or a multiple emulsion (W/O/W or polyol/O/W or O/W/O), in the form of a cream, a paste, a mousse, a dispersion of vesicles, especially of ionic or nonionic lipids, a two-phase or multiphase lotion, a spray, a powder, a paste, especially a soft paste or an anhydrous paste, a stick or a cast solid.

12. Cosmetic composition according to any one of Claims 1 to 11, **characterized in that** it is in anhydrous form.

13. Composition according to one of Claims 1 to 11, **characterized in that** it is a lip makeup product.

14. Cosmetic use of a composition according to one of Claims 1 to 12.

15. Process for making up and/or caring for the skin and/or the lips, comprising the application to the skin and/or the lips of at least one composition according to any one of Claims 1 to 12.

**Patentansprüche**

1. Kosmetische Schmink- und/oder Pflegezusammensetzung der Haut und/oder der Lippen, umfassend mindestens einen Ester von Alkoxylalkohol und Carbonsäure und mindestens einen von dem vorhergehenden Ester verschiedenen Kohlenwasserstoffester, wobei die Zusammensetzung dazu ausgelegt ist, ein Depot mit einem Beständigkeitsindex von größer oder gleich 30 % zu bilden, wobei der Ester von Alkoxylalkohol und Carbonsäure ausgewählt ist aus Octyldocecyl-PPG-3-myristyletherdilinoleat, Stearyl-PPG-3-myristyletherdilinoleat und Isostearyl-PPG-4-butyloctyletherdilinoleat, wobei der Kohlenwasserstoffester ein Monoester ist, der ausgewählt ist aus Neopentansäureestern, wie Isodecylneopentanoat, Isotridecylneopentanoat, Isostearylneopentanoat und Octyldocecylneopentanoat, Isononansäureestern, wie Isononylisononanoat, Octylisononanoat, Iosdecylisononanoat, Isotridecylisononanoat und Isostearylisononanoat, Oleylerucat und ihren Gemischen.

2. Zusammensetzung nach Anspruch 1, **dadurch gekennzeichnet, dass** der Monoester ausgewählt ist aus Octyldocedylneopentanoat, Isononylisononanoat, Oleylerucat und ihren Gemischen.

3. Zusammensetzung nach Anspruch 1 oder 2, **dadurch gekennzeichnet, dass** der Alkoxylester Octyldocecyl-PPG-3-myristyletherdilinoleat ist.

4. Zusammensetzung nach Anspruch 1 oder 2, **dadurch gekennzeichnet, dass** der Alkoxylester Isostearyl-PPG-4-butyloctyletherdinlinoleat ist.

5. Zusammensetzung nach einem der vorhergehenden Ansprüche, **dadurch gekennzeichnet, dass** der Kohlenwasserstoffester so ist, dass wenn er in einer ausreichenden Menge in der Zusammensetzung vorhanden ist, die Zusammensetzung dazu ausgelegt ist, ein Depot zu bilden, dessen Beständigkeit größer oder gleich 30 % ist.

6. Zusammensetzung nach einem der vorhergehenden Ansprüche, **dadurch gekennzeichnet, dass** sie dazu ausgelegt ist, ein Depot mit einem Beständigkeitsindex von größer oder gleich 40 % zu bilden.

7. Zusammensetzung nach dem vorhergehenden Anspruch, **dadurch gekennzeichnet, dass** sie dazu ausgelegt ist, ein Depot mit einem Beständigkeitsindex von größer oder gleich 50 %, sogar über 60 %, sogar über oder gleich 65 % zu bilden.

**8.** Zusammensetzung nach einem der vorhergehenden Ansprüche, **dadurch gekennzeichnet, dass** sie weiterhin einen oder mehrere Farbstoffe umfasst, ausgewählt aus wasserlöslichen oder fettlöslichen Farbstoffen und feinpulvrigen Farbstoffen, wie Pigmente, Lacke und Pailletten.

**9.** Zusammensetzung nach einem der vorhergehenden Ansprüche, **dadurch gekennzeichnet, dass** sie weiterhin mindestens einen Fettkörper umfasst, ausgewählt aus Wachsen, pastösen Fettkörpern und ihren Gemischen.

**10.** Zusammensetzung nach einem der vorhergehenden Ansprüche, **dadurch gekennzeichnet, dass** sie einen kosmetischen Bestandteil umfasst, ausgewählt aus Vitaminen, Verdickern, Gelbildnern, Obligo-Elementen, Süßungsmitteln, Sequestriermitteln, Duftstoffen, alkalisierenden oder acidifizierenden Mitteln, Konservierungsstoffen, Sonnenschutzmitteln, grenzflächenaktiven Mitteln, Antioxidantien, Fasern oder ihren Gemischen.

**11.** Kosmetische Zusammensetzung nach einem der vorhergehenden Ansprüche, **dadurch gekennzeichnet, dass** sie in Form von Suspension, Dispersion, Lösung, Gel, Emulsion, insbesondere Öl-in-Wasser- (H/E) oder Wasser-in-Öl- (E/H), oder Mehrfachemulsion (E/H/E oder Polyol/H/E oder H/E/H), in Form von Creme, Paste, Schaum, Dispersion von Vesikeln, insbesondere ionischen oder nichtionischen Lipiden, zwei- oder mehrphasiger Lotion, Spray, Pulver, Paste, insbesondere von weicher Paste oder wasserfreier Paste, in Stickform oder in Form eines gegossenen Festkörpers vorliegt.

**12.** Kosmetische Zusammensetzung nach einem der Ansprüche 1 bis 11, **dadurch gekennzeichnet, dass** sie in wasserfreier Form vorliegt.

**13.** Zusammensetzung nach einem der Ansprüche 1 bis 11, **dadurch gekennzeichnet, dass** es sich um ein Schminkprodukt für die Lippen handelt.

**14.** Kosmetische Verwendung einer Zusammensetzung nach einem der Ansprüche 1 bis 12.

**15.** Verfahren zum Schminken und/Pflegen der Haut und/oder der Lippen, umfassend das Aufbringen auf die Haut, die Lippen von mindestens einer Zusammensetzung nach einem der Ansprüche 1 bis 2.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20020192249 A
- US 5693316 A
- US 6476254 B
- WO 2003013439 A
- US 5302377 A
- US 5455025 A
- US 5597555 A
- WO 052076 A
- FR 0209246
- EP 0955039 A
- FR 0304259